# FASCICULE DE BREVET EUROPEEN

(11) **EP 0 705 291 B1**
(45) Date de publication et mention de la délivrance du brevet: **10.09.2003**
(21) Numéro de dépôt: 95918636.2
(22) Date de dépôt: 24.04.1995
(51) Int. Cl.: C08G 61/12, G01N 27/00

(54) **POLYMERES CONJUGUES FONCTIONNALISES ELECTRIQUEMENT CONDUCTEURS ET ELECTROACTIFS, ET UTILISATIONS**
ELEKTRISCH LEITFÄHIGE UND ELEKTROAKTIVE KUNJUGIERTE UND FUNKTIONNALISIERTE POLYMERE UND IHRE ANWENDUNG
ELECTRICALLY CONDUCTIVE ELECTROACTIVE FUNCTIONALISED CONJUGATED POLYMERS, AND USES THEREOF

(30) Priorité: 22.04.1994 FR 9405064
(43) Date de publication de la demande: 10.04.1996
(73) Titulaire: BIO MERIEUX, 69280 Marcy l'Etoile (FR)
(72) Inventeur: GARNIER, Francis, F-94500 Champigny-sur-Marne (FR)
(74) Mandataire: Guerre, Dominique
(86) Numéro de dépôt international: FR9500536
(87) Numéro de publication internationale: WO95029199

(56) Documents cités:
- EP-A- 0 157 326
- WO-A-90/10655
- WO-A-93/06237
- FR-A- 2 656 868

## Description

Les polymères conjugués, tels que polypyrroles, polythiophènes, polyanilines, polyphénylènes, et leurs dérivés sont connus pour leur caractère électroactif, largement décrit dans des ouvrages de revue tel que "Handbook of Organic Conducting Polymers (T.J. Skotheim Editeur, Marcel Dekker, New York, 1986). Ces polymères sont obtenus sous forme de film sur électrode, sous forme de films auto-supportés ou encore sous forme de composite lorsqu'alliés à un polymère polycationique ou polyanionique et se comportent comme des électrodes organiques, qui se chargent suivant un processus d'oxydation anodique, par insertion d' ions du milieu électrolytique. Ce processus électrochimique est réversible, la réduction provoquant l'expulsion des ions de ce polymère conjugué ou du composite électroactif.

Une seconde génération de polymères conjugués a ensuite été décrite dans la littérature, obtenue par le greffage covalent, sur les unités monomères des polymères, de groupes fonctionnels, capables d'apporter une fonction additionnelle à ces polymères conjugués électroactifs. A titre d'exemple, des complexes métalliques électrocatalytiques ont été greffés sur les unités monomères du polypyrrole, des macrocycles complexants spécifiques ont été greffés sur les chaines de polypyrrole ou de polythiophène pour la reconnaissance de cations dans un milieu électrolytique, des groupes chiraux ont été greffés sur des polythiophènes pour la reconnaissance d'anions optiquement actifs. L'ensemble de ces voies de fonctionnalisation a fait également l'objet de mises au point détaillées dans la littérature (F. Garnier, Angew. Chemie, 1989, 101, 529; A. Deronzier, J.C. Moutet, Acc. Chem. Res. 1989, 22, 249; J. Roncali, Chem Rev., 1992, 92, 711).

Ces dernières années des auteurs se sont intéressés à l'utilisation de polymères conducteurs fonctionnalisés pour le développement de capteurs d'analytes, notamment à but diagnostique. Toutefois, comme indiqué dans la demande de brevet EP 0 314 009, il était communément admis par la communauté scientifique que des polymères de pyrrole substitués au niveau soit de l'atome d'azote , soit directement au niveau des atomes de carbone du cycle pyrrole n'étaient pas de bons candidats pour le développement de capteurs d'analytes du fait notamment de la perte de conductivité desdits polymères quand des groupements fonctionnels sont introduits sur le cycle hétéroatomique. Pour résoudre ce problème les auteurs de cette demande de brevet avaient donc envisagé d'utiliser des polymères 2,5-di(2-thienylpyrrole) sur lesquels était greffée en position 3 du noyau pyrrole une partie réactive à laquelle pouvait être liée par covalence une molécule organique. Il convient toutefois de noter qu'en raison de l'hydrophobicité des noyaux thiophènes les polymères décrits ne peuvent être conducteurs et électroactifs dans les milieux aqueux et de ce fait ne semblent être adaptés pour la détection et/ou la caractérisation d'un analyte dans un échantillon biologique (voir J. Roncali et al., Chem. Comm., 1986, page 783 et G. Tourillon et al., Electronal.Chem., 161, 407, 1984).

On a maintenant découvert de manière tout à fait surprenante et à l'encontre de ce qui était jusque là admis par les spécialistes que la conductivité et l'électroactivité de polypyrroles sont conservées à la condition que le greffage d'un groupement fonctionnel soit effectué en position 3 ou 4 sur le noyau pyrrole à l'aide d'un agent de fonctionnalisation qui permet l'éloignement de la fonction visée par rapport au noyau pyrrole. A l'extrémité libre du groupement fonctionnel est lié par covalence un anti-ligand sans que soient modifiées les propriétés précitées du polymère. De tels polymères fonctionnalisés n'ont à ce jour jamais été décrits et se sont révélés être parfaitement appropriés comme capteurs d'un ligand biologique. Par ailleurs, les polypyrroles se révèlent être des polymères intéressants du fait de leur biocompatibilité. Enfin, les polypyrroles ainsi fonctionnalisés permettent de réaliser des polymères électroactifs et conducteurs d'épaisseurs importantes (jusqu'à plusieurs millimètres), ce qui autorise une grande densité de sites fonctionnels et améliore d'autant la sensibilité.

L'invention a donc pour *premier* objet un polymère fonctionnalisé, électriquement conducteur et électroactif qui répond à la formule I : 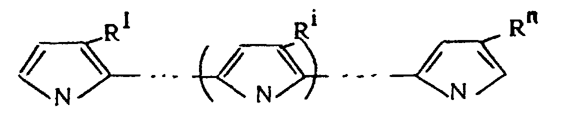 dans laquelle:
* n est un nombre entier non nul et i est un nombre entier variant de 2 à n-1, et
* R¹, Rⁱ, Rⁿ, identiques ou différents, représentent H ou un groupe fonctionnel *à l'exclusion de CH*_{*2*}*-COOH*, susceptible de se lier, par covalence, à une première molécule biologique ou anti-ligand, et en ce que ledit polymère présente une conductivité et une électroactivité sensiblement du même ordre que celle du polymère conjugué non fonctionnalisé correspondant, c'est-à-dire du polymère de formule I correspondant, dans laquelle R¹, Rⁱ, Rⁿ représentent chacun H.

Plus particulièrement, le ou les groupement(s) fonctionnel(s) sont indépendamment choisis dans l'ensemble de groupes fonctionnels suivants :
Yₚ-C-X où X représente H, OH, un radical O-alkyle inférieur substitué ou non, un halogène, notamment Cl ; Yₚ-NHZ, Z représentant H ou un radical alkyle ; Yₚ-NH-CO-CF₃ ; Yₚ-X où X répond à la définition ci-dessus, p étant un nombre entier de préférence égal à 0, 1 ou 2 ; -Si(alkyle)₃, -Si(alkoxyle)₃, ou un groupement ester activé tel que le N-hydroxy-succinimide.

Y représente un groupement choisi parmi les alkyles ayant de 1 à 5 atomes de carbone, les alkoxyles ayant de 1 à 5 atomes de carbone, les polyéthers répondant à la formule générale (CH₂-CH₂-O)ₘ-(CH₂)_{m'}-, m représentant un nombre entier variant de 1 à 3 et m' un nombre entier égal à 1 ou 2.

L'invention a pour *second* objet un polymère conjugué, électriquement conducteur, et électroactif comprenant au moins un groupe fonctionnel lié, par covalence, à une première molécule biologique ou anti-ligand répondant à la formule II : 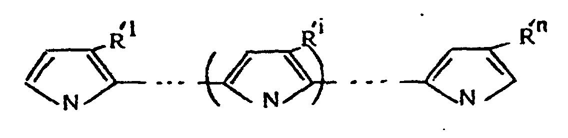 dans laquelle :
* n est un nombre entier non nul et i est un nombre entier variant de 2 à n-1, et
* R'¹, R'ⁱ, R'ⁿ, identiques ou différents, représentent chacun H ou un groupe fonctionnel susceptible de se lier, ou lié, par covalence, à une première molécule biologique ou anti-ligand.

Les groupes fonctionnels liés à une molécule biologique ou ligand sont choisis, avant réaction avec cette dernière, dans l'ensemble des groupes fonctionnels suivant :
Yₚ-C-X où X représente H, OH, un radical O-alkyle inférieur substitué ou non, un halogène, notamment Cl ; Yₚ-NHZ, Z représentant H ou un radical alkyle ; Yₚ-NH-CO-CF₃ ; Yₚ-X où X répond à la définition ci-dessus, p étant un nombre entier de préférence égal à 0, 1 ou 2 ; -Si(alkyle)₃, -Si(alkoxyle)₃, ou un groupement ester activé tel que le N-hydroxy-succinimide.

En particulier, les groupes fonctionnels liés à une molécule biologique sont identiques et consistent, avant réaction avec la ou les première(s) molécule(s) biologique(s), en -(CH₂)-COOH, lesdites premières molécules biologiques ou anti-ligands étant choisies parmi les peptides ou dérivés de peptides notamment Gly-Phe, Phe-Pro, Phe-HEA-Pro, et parmi les polynucléotides tels que l'oligonucléotide de séquence: CCTAAGAGGGAGTG.

Un *troisième* objet de l'invention est l'utilisation d'un polymère conjugué tel que défini précédemment *selon le second objet de l'invention,* pour détecter ou doser, in vitro ou in vivo, une seconde molécule biologique ou ligand, différente de l'anti-ligand et interagissant spécifiquement avec ce dernier, la détection et/ou le dosage dudit ligand étant effectué par observation et/ou mesure d'une différence de potentiel ou d'une variation de courant entre le polymère conjugué non lié au ligand et le polymère conjugué lié au ligand.

En particulier, les polymères de l'invention sont utilisés pour détecter et/ou doser une enzyme, telle qu'une enzyme protéolytique notamment la carboxypeptidase A, ou un polynucléotide ou pour extraire, in vitro ou in vivo, une seconde molécule biologique ou ligand, différente de l'anti-ligand et interagissant spécifiquement avec ce dernier.

Dans un mode de réalisation de l'invention, le polymère conjugué est déposé sur un substrat conducteur, tel que le métal ou un dérivé du carbone, ou sous la forme d'un film auto-supporté.

Enfin, l'invention concerne une électrode et un film auto supporté constitués d'un substrat conducteur tel qu' un métal ou un dérivé de carbone et d'un polymère tel que défini précédemment, *selon le second objet de l'invention.*

Dans un mode de réalisation, l'anti-ligand est spécifique du ligand ou molécule cible. L'anti-ligand est notamment choisi pour former un complexe anti-ligand/molécule cible. A titre d'exemple, le complexe peut être notamment représenté par tout couple peptide/anticorps, anticorps/haptène, hormone/récepteur, les hybrides polynucléotide/polynucléotide, polynucléotide/acide nucléique ou analogues.

Le terme "polynucléotide" tel qu'utilisé dans la présente invention désigne un enchaînement d'au moins cinq désoxyribonucléotides ou ribonucléotides comprenant éventuellement au moins un nucléotide modifié, par exemple un nucléotide comportant une base modifiée telle que l'inosine, la méthyl-5-désoxycitidine, la diméthylamino-5-désoxyuridine, la désoxyuridine, la étamine-2,6-purine, la bromo-5-désoxyuridine ou tout autre base modifiée permettant l'hybridation. Ce polynucléotide peut également être modifié au niveau de la liaison internucléotidique (comme par exemple les liaisons phosphorothioate, H-phosphonate, alkyl-phosphonate), au niveau du squelette comme par exemple les alpha-oligonucléotides (FR 2 607 507) ou les PNA (M. Egholm et al., J. Am. Chem. Soc., (1992), 114, 1895-1897). Chacune de ces modifications peut être prise en combinaison.

Le terme "peptide" tel qu'utilisé dans la présente demande signifie notamment tout peptide d'au moins deux acides aminés, notamment protéine ou fragment de protéine, oligopeptide, extrait, séparé ou substantiellement isolé ou synthétisé, notamment ceux obtenus par synthèse chimique ou par expression dans un organisme recombinant; tout peptide dans la séquence duquel un ou plusieurs acides aminés de la série L sont remplacés par un acide aminé de la série D, et vice-versa; tout peptide dont l'une au moins des liaisons CO-NH, et avantageusement toutes les liaisons CO-NH de la chaîne peptidique est (sont) remplacée(s) par une (des) liaisons NH-CO; tout peptide dont l'une au moins des liaisons CO-NH et avantageusement toutes les liaisons CO-NH est ou sont remplacée(s) par une ou des liaison(s) NH-CO, la chiralité de chaque résidu aminoacyle, qu'il soit impliqué ou non dans une ou plusieurs liaisons CO-NH sus-mentionnées, étant soit conservée, soit inversée par rapport aux résidus aminoacyles constituant un peptide de référence, ces composés étant encore désignés immunorétroïdes, un mimotope.

De nombreuses classes de peptides peuvent être greffées, comme le montre la liste non exhaustive ci-après: hormones adrénocorticotropiques ou leurs fragments; analogues d'angiotensines ou leurs inhibiteurs (composants du système renine-angiotensine qui régulent l'hypertension rénale); peptides natriurétiques; bradykinine et ses dérivés peptidiques; peptides chimiotactiques; dynorphine et ses dérivés; endorphines ou analogues; encéphalines ou leurs dérivés; inhibiteurs d'enzymes (tels que protéases); fragments de fibronectine et dérivés; peptides gastrointestinaux; peptides associés au largage d'hormones de croissance; neurotensines et analogue; peptides opioides; oxytocine, vasopressine, vasotocine et dérivés; protéines kinases.

Les peptides ou les polynucléotides possèdent une activité biologique élevée, et sont connus pour contrôler de nombreuses fonctions biologiques (A.S. Dutta, Advances in Drug Research, B. Testa Editeur, Academic Press, New York, 1991, 21, 145). Les peptides montrent par exemple un potentiel thérapeutique très important en tant que récepteur agoniste ou antagoniste, en tant qu' inhibiteurs très puissants qui se lient de façon forte à des enzymes, principe sur lequel est basée la chromatographie dite d'affinité. Par ailleurs, les polynucléotides peuvent, par réaction d'hybridation sélective avec d'autres nucléotides ou fragments d'acides nucléiques cibles, donner lieu à des phénomènes de reconnaissance intéressants, permettant notamment le développement de nouveaux capteurs de gènes. Ainsi, pour détecter et/ou doser un acide nucléique ou un fragment d'acide nucléique cible, un polymère fonctionnalisé lié au moins partiellement à un polynucléotide antiligand est mis en contact avec un échantillon susceptible de contenir la cible, puis on détecte la réaction d'hybridation si elle a eu lieu, soit directement par mesure d'une différence de potentiel ou de variation de courant entre le polymère non lié et le polymère lié ayant réagi avec la cible, soit indirectement par la même mesure que précédemment, mais à l'aide d'un polynucléotide supplémentaire de détection susceptible de réagir avec la cible, ledit polynucléotide supplémentaire étant de préférence contigu au polynucléotide antiligand et marqué avec une molécule électroactive.

Le terme "anticorps" tel qu'utilisé dans la présente demande signifie tout anticorps monoclonal ou polyclonal, tout fragment d'undit anticorps tel que fragment Fab, Fab'2 ou Fc, ainsi que tout anticorps obtenu par modification ou recombinaison génétique.

La fonctionnalisation du polypyrrole en position 3 ou 4 du cycle pyrrole peut être effectuée, soit sur les unités monomères avec étape de polymérisation subséquente, soit sur les unités monomères d'un polymère synthétisé au préalable. Tout agent de fonctionnalisation approprié peut être utilisé, à la condition qu'il comprenne au moins une fonction réactive susceptible de réagir avec les atomes 3 et/ou 4 du noyau pyrrole. L'agent de fonctionnalisation peut ainsi être un agent unifonctionnel, à la condition qu'après l'étape de greffage sur le noyau pyrrole une nouvelle fonction réactive soit introduite pour réaction subséquente avec l'anti-ligand, soit plurifonctionnel tel que des agents bifonctionnels et en particulier homo ou hétéro bifonctionnel. A titre d'exemple, l'agent de fonctionnalisation est choisi parmi les chaines alkyles ou alkoxyles ou polyéthers substituées ou non et terminées par un groupe portant une fonction réactive. La fonction réactive est notamment représentée par un groupement fonctionnel tel que groupement carboxylique, hydrazide, amine, nitrile, aldéhyde, thiol, disulfure iodoacétyle, ester, anhydride, tosyle, mésyle, trityle, silyle ou analogues.

La formation d'un conjugué résultant du couplage covalent d'un anti-ligand, par exemple un polynucléotide à un polypyrrole fonctionnalisé selon l'invention peut être effectuée selon les méthodes dites directes ou indirectes connues.

Par exemple, dans le cas d'un polynucléotide, selon la méthode directe, on synthétise un polynucléotide ayant une fonction réactive sur un site quelconque de la chaîne nucléotidique comme par exemple, l'extrémité 5' ou l'extrémité 3', ou sur une base ou sur un phosphate internucléotidique, ou sur la position 2' d'un sucre. Le polynucléotide est ensuite couplé au polymère, préparé au préalable et comportant une fonction réactive complémentaire de la précédente, c'est-à-dire permettant la formation d'une liaison covalentc par réaction entre les deux fonctions réactives complémentaires, l'une portée par le polynucléotide et l'autre par le polymère fonctionnalisé. Par exemple, de façon connue, on peut coupler des amines primaires avec un acide carboxylique activé ou un aldéhyde ou bien une fonction thiol avec un halogénoalkyle. De préférence, la fonction réactive du polynucléotide pour le couplage sur le polymère est à l'extrémité 5' ou 3'.

Dans la méthode de couplage indirecte, le polynucléotide et le polymère sont chacun porteurs d'une fonction réactive, ces fonctions réactives pouvant être identiques ou différentes l'une de l'autre, ces deux fonctions n'étant pas complémentaires mais étant capables de réagir avec un agent intermédiaire de couplage qui est un réactif bifonctionnel (homobifonctionnel si les deux fonctions sont identiques ou hétérobifonctionnel si les deux fonctions sont différentes). Parmi les agents de couplage homobifonctionnels, on peut citer le DITC (phénylène-1,4-diisothiocyanate), le DSS (disuccinimidylsubérate) ou analogues. Parmi les agents de couplage hétérobifonctionnels, on peut citer le SMCC (succinimidyl-4-(N-maléimidométhyl) cyclohexane-1-carboxylate), ou le SMPB (succinimidyl-4-(p-maléimido phényl) butyrate), capables de réagir d'une part avec une amine primaire et d'autre part avec un thiol.

L'invention sera mieux comprise à la lecture de la description détaillée qui va suivre faite en référence aux figures annexées dans lesquelles,

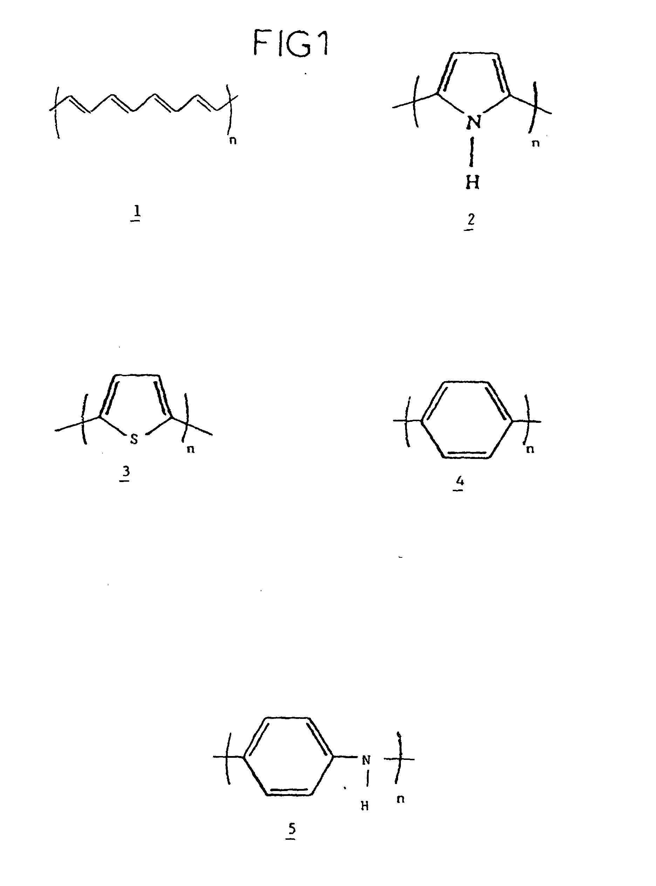

la figure 1 représente des exemples de polymères conjugués tels que 1) polyacétylène: 2) polypynrole ; 3) polythiophène ; 4) polyphénylène ; 5) polyaniline ;
la figure 2A représente un polypyrrole substitué en position 3 avec l'acide acétique (1) et les figures 2B, 2C, 2D, 2E et 2F représentent des polypyrroles substitués en position 3 avec différents peptides (2 à 6, respectivement) ,
la figure 3 représente les voltamogrammes de quatre polymères fonctionnalisés, ci-dessous identifiés en milieu H₂O-NaCl 0,5 M
   le poly(pyrrole-acétique acide) en (1), le poly(pyrrole(Gly-DPhe)) en (2), le poly(pyrrole(Val)) en (3) et le poly(pyrrole(Phe)) en (4) ;
la figure 4 représente le voltamogramme de Poly(2), en milieu H₂O-NaCl 0,5 M, en présence de carboxypeptidase A en concentration respectivement de 0,0 mg dans 5cm³ d'électrolyte (a), 1,2 mg dans 5cm³ d'électrolyte (b), 2,4 mg dans 5cm³ d'électrolyte (c), et 5,0 mg dans 5cm³ d'électrolyte (d) ;
la figure 5 correspond à la réponse ampérométrique d'une électrode, Poly(2), en fonction de la quantité d' enzyme, carboxypeptidase A, en nanomoles, existant dans le milieu. La relation linéaire entre courant observé et quantité d'enzyme, à un potentiel de 0,3 V est donnée par rapport à une électrode calomel saturé,
la figure 6, est un schéma de principe d'un transistor microélectrochimique à effet de champ pour la détection (amplifiée) de la présence d'une espèce biologique reconnue par un polymère conducteur fonctionnalisé. Les abréviations suivantes signifient respectivement: P, polymère. Sub, substrat. S, D, électrodes de source et de drain respectivement. CE, contre-électrode jouant le rôle de grille G. R, électrode de référence. Poten., potentiostat;
la figure 7 est un schéma de principe d'une cellule électrochimique à membrane polypyrrole fonctionnalisée et à deux compartiments, pour l'extraction d'espèces biologiques reconnues par un substituant greffé sur une chaîne de polymère conjugué électroactif;
la figure 8 représente le voltamogramme du poly[N-hydroxysuccinimide-3-pyrrole], en milieu acétonitrile-0,1 M LiClO₄ avec une électrode de référence au calomel saturé, montrant une électroactivité et une réversibilité électrochimique élevées;
la figure 9 représente le voltamogramme d'une électrode de copolymère Poly([pyrrole-ODN][pyrrole-COON]), avec comme polynucléotide ou oligonucléotide ODN de séquence CCTAAGAGGGAGTG. Aucune modification n'est observée après incubation de ce polymère avec une séquence non cible, GGTGATAGAAGTATC; et
la figure 10 représente le voltamogramme d'une électrode de copolymère Poly([pyrrole-ODN][pyrrole-COOH]), avec comme oligonucléotide ODN: CCTAAGAGGGAGTG. Cette électrode a été incubée en présence d'une cible, CACTCCCTCTTAGG, 335 nmole, à 37°C pendant 2h. Cette électrode a ensuite été rincée et analysée en milieu électrochimique. Un déplacement de potentiel est observé par rapport au voltamogramme précédent.

Les polymères selon l'invention sont notamment utilisables pour la détection d'espèces biologiquement actives susceptibles d'être présentes dans un échantillon et de réagir avec l'anti-ligand ou les anti-ligands greffés. En effet, comme montré ci-après on observe que les polymères conjugués fonctionnalisés en position 3 de leur hétérocycle et auxquels sont greffés un ou plusieurs anti-ligands, après réaction avec un ou plusieurs ligands, présentent une modification de la réponse électrochimique par rapport à un polymère de référence n'ayant pas réagi avec le ou les ligands d'un milieu biologique, visualisée par un changement du potentiel d'oxydation. Cette variation du voltamogramme électrochimique d'oxydoréduction du polymère confère une fonction de capteur, et peut ainsi être utilisée pour une mesure quantitative de l'espèce biologiquement active, soit par la variation du potentiel, à courant fixe, soil par la variation du courant, à potentiel fixe, ou encore par la réalisation de transistors microélectrochimiques à effet de champ.

Par ailleurs, les polymères de l'invention sont également utilisables pour l'extraction d'espèces biologiquement actives en solution. Dans plusieurs cas, l'espèce biologiquement active en solution se combine de façon forte à l'anti-ligand greffé sur la chaîne de polymère, tel qu'un peptide bioactif ou un polynucléotide, ce qui permet donc d'extraire l'espèce biologiquement active de façon sélective d'un milieu. Ce type d'extraction peut être réalisé *in vitro*, ou même *in vivo* lorsque le polymère support est biocompatible, tel que le polypyrrole par exemple.

Enfin, les polymères de l'invention peuvent être une source de relargage, d'un milieu dans un autre milieu, d'espèces biologiquement actives (enzymes ou autres).

La fonctionnalisation des polymères conducteurs électroactifs de l'invention, tels que polypyrroles, par des groupes montrant une reconnaissance vis-à-vis de composés d'intérêt biologique peut être étendue à la reconnaissance d'acides nucléiques (AN). Ainsi le greffage de polynucléotides ou oligonucléotides, ODN, le long de la chaîne conjuguée de polymères doit permettre la discrimination au sein d'un milieu biologique des AN ou fragments d'AN correspondants. Cette reconnaissance sera opérée par hybridation sélective entre l'ODN, greffé sur le polymère, et l'AN correspondant existant dans le milieu externe, dans lequel est immergé le film de polymère fonctionnalisé, à l'image de la reconnaissance "peptide/enzyme" décrite ultérieurement. La complexation "ODN/AN" résulte en une modification des propriétés physicochimiques du polymère conjugué, dont la caractérisation permettra de confirmer la présence de l'AN recherché.

Le point essentiel concerne la nature des propriétés physicochimiques du polymère appelées à être modifiées lors de la reconnaissance "ODN/AN". En effet, afin de développer une méthode de mesure rapide, sensible et quantitative de la présence d'AN, le but de la présente invention concerne l'élaboration de matériaux électroactifs, dont la réponse électrochimique sera modifiée après hybridation "ODN/AN". La modification concernera une variation de type potentiométrique, comme variation du potentiel d'oxydation du polymère, ou ampérométrique, par variation du courant d'oxydation (ou de réduction) observé à un potentiel déterminé. Ces variations de réponse électrochimique pourront être mesurées quantitativement, les films de polymères fonctionnalisés étant utilisés soit comme capteurs électrochimiques de type ampérométrique ou potentiométrique, soit encore dans une structure de transistor microélectrochimique à effet de champ, ainsi que cela a été décrit précédemment dans le cas de la reconnaissance enzymatique à partir de peptides greffés sur polypyrrole. L'intérêt de ce type de mesures concerne la rapidité, la sensibilité, et la possibilité de réaliser aisément des cartes matricielles de 2n éléments de mesure, comportant n ODN cibles et non cibles, capables donc de discriminer rapidement la présence ou l'absence de gènes dans un milieu.

De même que dans le cas de la reconnaissance enzymatique précédemment décrite, un second point essentiel concerne le fait que pour obtenir une réponse électrochimique à un phénomène de reconnaissance, la fonctionnalisation en position 3 d'un noyau hétérocyclique (pyrrole) est indispensable.

Afin d'assurer une réponse de type électrochimique précise pour ces polymères, il est nécessaire que la fonctionnalisation des chaînes conjuguées soit compatible avec une électroactivité importante du polymère fonctionnalisé. La nécessité d'une telle électroactivité exige, dans le cas de polyhétérocycles hydrophiles tels que le polypyrrole, que la fonctionnalisation soit opérée en position 3 du cycle pyrrolique. Les polymères de l'invention sont des polymères électroactifs dans lesquels soit toutes les unités monomères sont fonctionnalisées avec un antiligand tel qu'un oligonucléotide, soit une partie seulement des unités monomères sont ainsi fonctionnalisées. Il est bien entendu que les unités monomères peuvent être fonctionnalisées par des antiligands identiques ou différents, dans ce dernier cas les polymères de l'invention peuvent être utilisés pour la détection de plusieurs ligands cibles dans un même échantillon. Les polymères de l'invention peuvent être réalisés par les différentes voies suivantes:
a) Polymères totalement fonctionnalisés
   Dans cette voie, la première étape concernera la fonctionnalisation du monomère, tel que pyrrole, par un antiligand tel qu'un oligonucléotide déterminé. La seconde étape concernera ensuite la polymérisation de ce monomère, aboutissant à un film de polymère dans lequel toutes les unités monomères sont fonctionnalisées.
b) Copolymères partiellement fonctionnalisés
   Dans le cas particulier des acides nucléiques cibles, et compte tenu du fait de leur taille généralement importante, la fonctionnalisation de toutes les unités monomères, de petites tailles, du polymère n'est pas nécessaire, et l'une des voies concernera donc la réalisation, d'un copolymère, qui fera intervenir d'une part les unités monomères fonctionnalisées décrites en a), et également des unités pyrrole non fonctionnalisées avec l'oligonucléotide antiligand.
c) Fonctionnalisation d'un polymère précurseur
   La fonctionnalisation partielle d'un film de polymère peut également être réalisée à partir d'un film de polymère conjugué, dans lequel sont introduits au préalable des groupes chimiques compatibles avec le greffage d'un antiligand tel qu'un oligonucléotide. Dans cette voie, un monomère comportant un synthon de greffage sera d'abord réalisé, tel que le [N-hydroxysuccinimide-3-pyrrole]. Le synthon [N-hydroxysuccinimide] est connu pour permettre le greffage ultérieur d'un oligonucléotide. Ce monomère sera par la suite polymérisé, ou encore copolymérisé avec un autre dérivé de pyrrole. Le film de polymère obtenu sera ensuite immergé dans un milieu réactionnel contenant un oligonucléotide, et la réaction de greffage de cet oligonucleotide sur les monomères pyrrole sera entreprise. Ce greffage n'interviendra en fait que sur une partie des unités monomères pyrrole constitutives du polymère.

### Exemple. 1: Synthèse des monomères

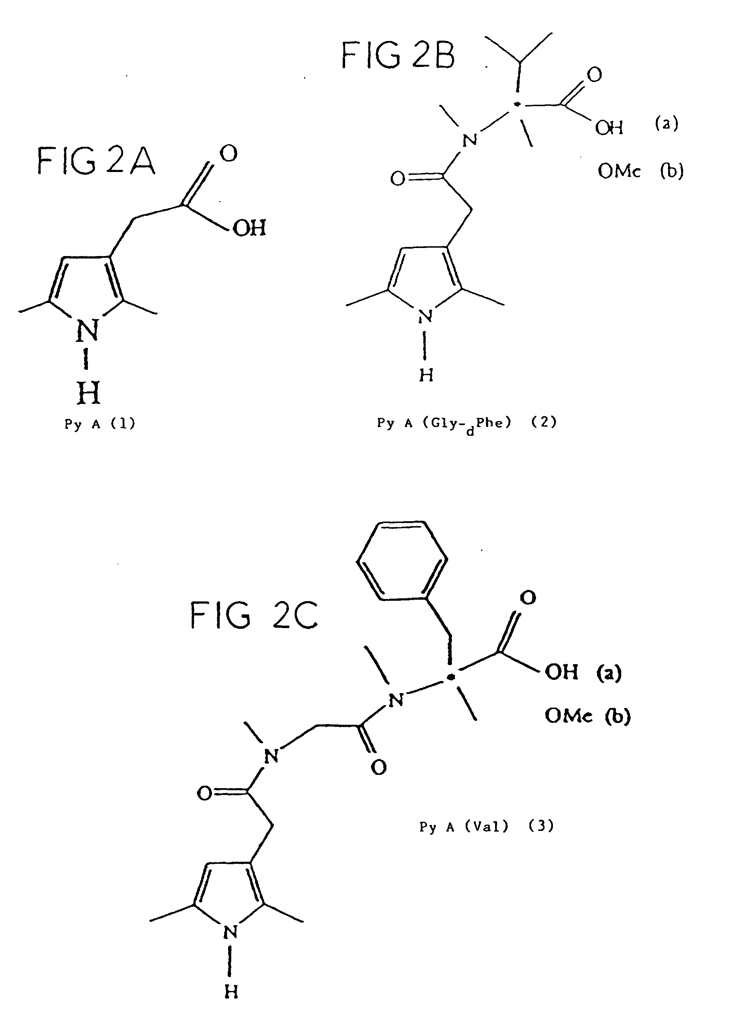

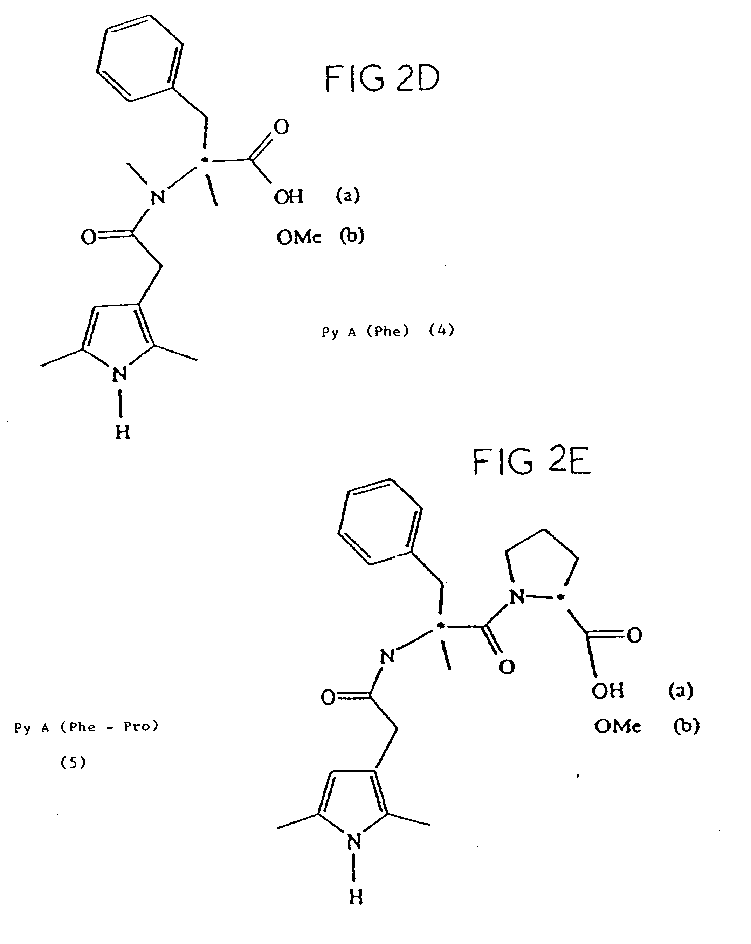

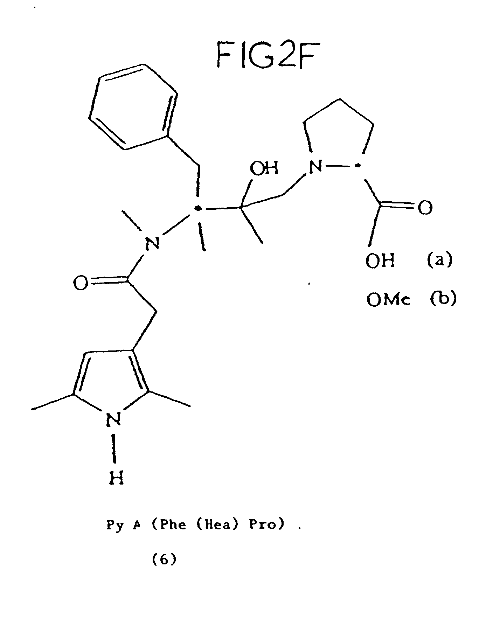

Dans l'exemple décrit dans ce qui suit, le polypyrrole (1) a été choisi comme support polymère conjugué compte tenu de sa biocompatibilité (H. Naarmann, communication personnelle). Un bras espaceur acétyle A, est greffé entre l'atome de carbone 3 du noyau pyrrole et le substituant peptidique pour préserver la conductivité et l'électroactivité du polypyrrole fonctionnalisé correspondant. Différents peptides, avec leur fonction terminale carboxylique non protégée ou protégée sous la forme ester méthylique, ont été choisis pour leur pertinence biologique et greffés sur un monomère pyrrole-acétique acide, PyA (1). Plusieurs mono et dipeptides ont été greffés, et ont conduit aux dérivés pyrroliques suivants, représentés sur la figure 2 : pyrrole-acétique acide, PyA (1), pyrrole(Glycine-dPhenylalanine), Py(Gly-DPhe) (2), pour sa capacité de complexation avec les enzymes protéolytiques tels que carboxypeptidase A (Sigma) et trypsine (Sigma) (J.R. Uren, Biochim. Acta, 1971, 236, 67), pyrrole(valine), Py(Val) (3), pyrrole(phénylalanine), Py(Phe) (4), pyrrole(Phénylalanine-Proline), Py(Phe-Pro).(5). Des dérivés de dipeptide plus volumineux peuvent également être greffés, tel que le Phénylalanine-Hydroxyethylamine-Proline, Py(Phe[HEA]Pro) (6), connu pour être un inhibiteur potentiel intéressant pour la protéase associée au virus HIV-1 du SIDA. Ces monomères ont été synthétisés suivant une voie chimique décrite (D. Delabouglise, F. Garnier, Synth. Met., 1990, 39, 117). Ces monomères ont été purifiés et caractérisés par RMN, microanalyse, et spectrométrie de masse.

### Exemple 2: Polymérisation

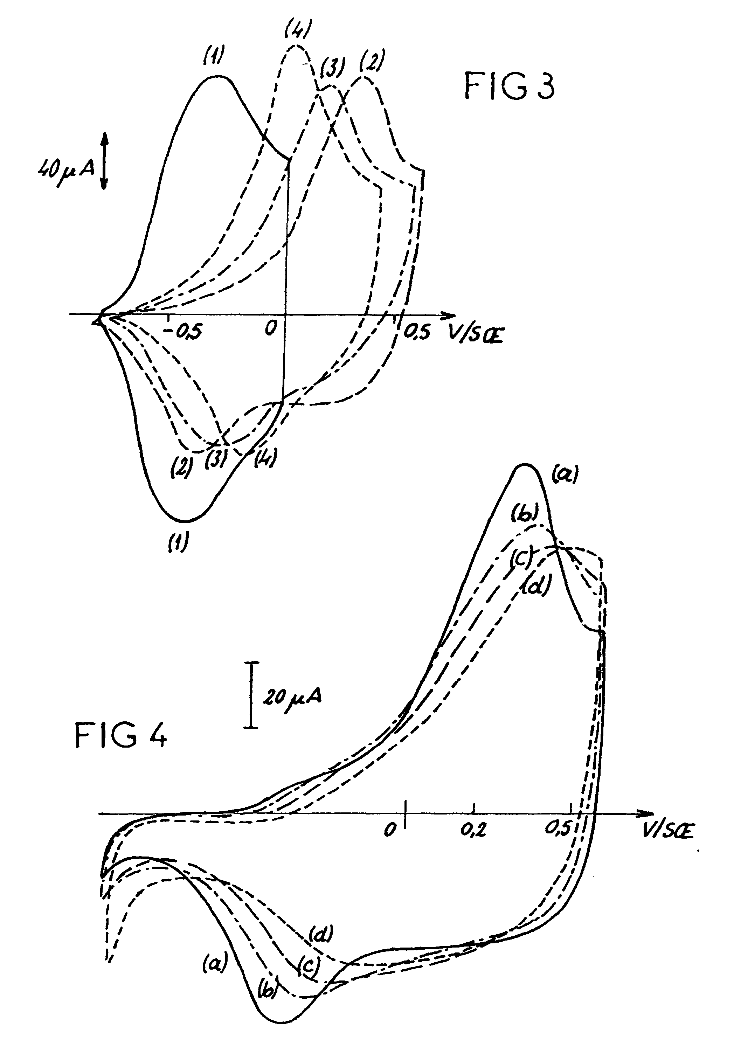

Ces monomères ont été polymérisés par voie électrochimique sur électrode de platine de 0,7 cm^{2,} ainsi que sur grille de platine de 10 cm² de surface, en milieu carbonate de propylène avec 0,5 M de NaCl, à un potentiel constant de 0,8 V/SCE. Des films épais de polymère sont obtenus, jusqu'à des épaisseurs de 10 µm. Comme le montre la figure 3, l'électroactivité de ces polymères a été confirmée par voltamétrie cyclique en milieu H₂O-NaCl 0,5 M, à un pH neutre de 7. Les valeurs de potentiel d'oxydation, de l'ordre de 0,30 V/SCE, proches de celle du polypyrrole non substitué, confirment l'électroactivité de ces polypyrroles fonctionnalisés avec des dipeptides.

### Exemple 3: Reconnaissance de carboxypeptidase A

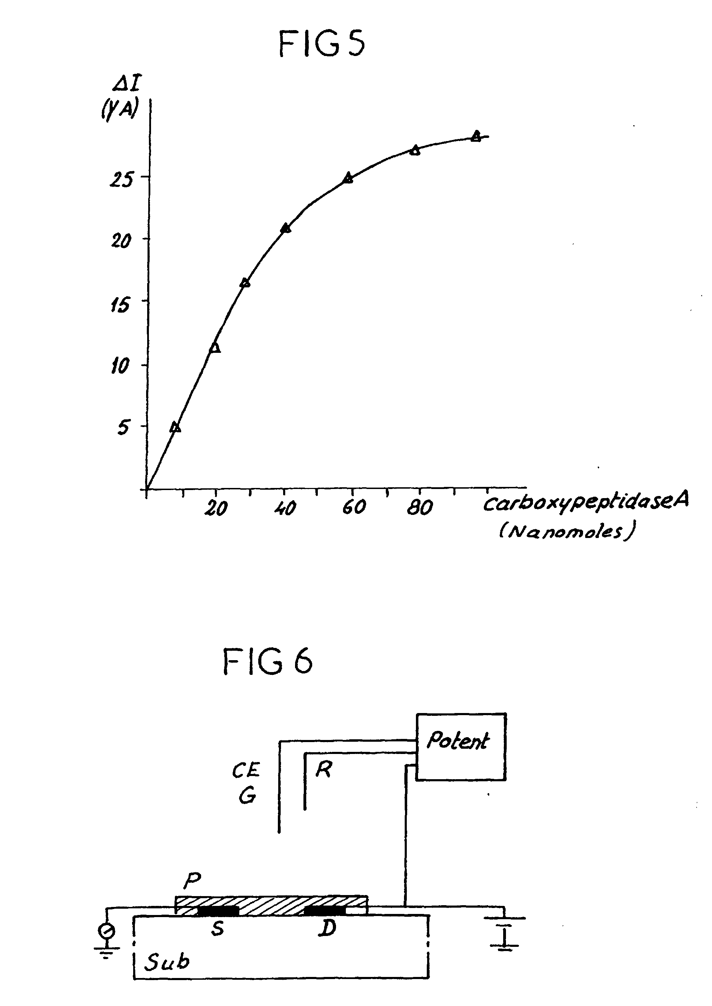

Les propriétés spécifiques de complexation de ces polypyrroles vis à vis d'enzymes protéolytiques ont été analysées avec la carboxypeptidase A, avec laquelle (Gly-DPhe) est connu pour former des complexes stables à pH neutre. Des solutions de concentration croissante de carboxypeptidase A, allant de 1mg à 5 mg dans 5 cm³ de H₂O-NaCl 0,5 M ont été analysées. Lorsque l'on immerge dans cette solution des électrodes non spécifiques telles que polypyrrole non substitué, ou poly(3, 4, 5 ou 6), un voltamogramme identique à celui obtenu à l'exemple 2 est observé, sans modification aucune. Lorsque cependant l'on utilise le poly(pyrrole(Gly-DPhe)), poly(2), le voltamogramme montre un déplacement vers les potentiels plus élevés, de 0,340 V/SCE pour une quantité initiale nulle en carboxypeptidase A jusqu'à une valeur limite de 0,500 mV/SCE pour une quantité de 5mg de carboxypeptidase A dans la solution. Ce résultat est reporté dans la figure 4, qui montre le déplacement de potentiel, attribué à un encombrement et une rigidification de la chaîne de polypyrrole, produite lors de la complexation de l'enzyme sur le dipeptide porté par la chaîne polymère. La formation de ce complexe entre enzyme et poly(pyrrole-dipeptide) a été confirmée par le relargage de l'enzyme en milieu acide, à pH = 3, ainsi que cela est réalisé classiquement en chromatographie d'affinité (I.M. Chaiken, M. Wilchek, I. Parikh, Affinity Chromatography and Biological Recognition, Academic Press, New York, 1983). L'enzyme relarguée a été caractérisée de façon classique par le test de Bradford, en mesurant l'activité enzymatique avec le bleu brillant de Comassii, et par l'utilisation d'un standard d'albumine de serum de bovin. Lorsque l'on utilise un film de poly(Gly-DPhe) contenant 5x10⁻⁶ unités monomères, correspondant à une charge de polymérisation de 1 Coulomb, une quantité significative de 400 microgrammes de carboxypeptidase A a été obtenue après relargage dans un milieu acide. Considérant la taille de cette enzyme, de 307 unités d'aminoacides, cette quantité d'enzyme relarguée montre qu'environ 1 molécule d'enzyme est complexée pour 200 unités monomères de (pyrrole-dipeptide), ce qui semble raisonnable compte tenu de la différence de taille (environ d'un facteur 100). Ce résultat montre également que l'enzyme doit être distribuée à l'intérieur du film de polymère, ce qui démontre sa perméabilité vis à vis de l'enzyme. Des résultats comparables ont été obtenus lorsque la trypsine a été utilisée comme enzyme.
A un potentiel déterminé de l'électrode, comme le montre la figure 4, une variation de courant est observée en fonction de la concentration d'enzyme. Ce résultat correspond à la réponse ampérométrique de l'électrode, et une des caractéristiques intéressantes de cette réponse concerne sa linéarité avec la concentration d'enzyme, tel que le montre la figure 5. Une telle relation linéaire permet de proposer un dosage quantitatif de l'espèce biologique en solution, soit par l'utilisation d'un capteur de type électrochimique, soit par le développement d'un transistor à effet de champ, représenté schématiquement en figure 6. Le principe de fonctionnement en est le suivant. Le polymère est réalisé sur les électrodes de source et de drain, déposées sur un substrat. Cet ensemble est immergé dans la solution à analyser, et une contre-électrode dans ce même milieu représente la grille de ce transistor. Lorsque cette électrode de grille est placée à un potentiel où la variation du voltamogramme en fonction de la concentration d'enzyme est maximale, vers 0,2 V dans le cas représenté à la figure 4, la conductivité du polymère varie de façon importante avec la concentration d'enzyme. Un potentiel appliqué alors entre électrodes de source et de drain permet d'obtenir un signal amplifié, ce transistor fonctionnant suivant le principe d'un transistor à effet de champ.

### Exemple 4: Relargage contrôlé d'une enzyme

Une caractéristique supplémentaire intéressante de ces électrodes concerne le fait qu'il a été montré dans la littérature que le poly(pyrrole acétique acide), ou poly(1) relargue des protons dans le milieu électrolytique lorsqu'il est soumis à oxydation électrochimique (P. Baüerle et al. Adv. Mater., 1990, 2, 490). Des variations importantes de pH peuvent être observées dans un faible volume électrolytique, jusqu'à des valeurs de l'ordre de 3. Ce relargage de protons s'observe également lorsque la fonction carboxylique est éloignée du noyau pyrrole, tel que c'est le cas pour un aminoacide ou un peptide non protégé greffé sur le pyrrole. Deux voies existent pour le relargage contrôlé de protons, soit l'utilisation d'un peptide dont la fonction terminale carboxylique n'est pas protégée, COOH, soit par l'utilisation d'un copolymère entre pyrrole-acétique acide et pyrrole-peptide protégé. Un exemple de cette deuxième voie est donné. Un copolymère entre Py(A), (1) et Py(Gly-DPhe), (2), a été réalisé électrochimiquement, dans les mêmes conditions que précédemment. Ce copolymère, Poly(1, 2) conduit, lorsque soumis à électrooxydation à 0,3 V/SCE à une variation de pH importante jusqu'à pH = 4 au voisinage de l'électrode.
A partir de ce copolymère Poly(1, 2), ou à partir du polymère Poly(2) dont la fonction carboxylique est libre, deux procédés peuvent être mis en oeuvre pour l'extraction, un procédé en discontinu et un procédé en continu.

### a) Procédé discontinu

Ce procédé consiste à utiliser une membrane ou électrode à base des matériaux précités, de la mettre en contact avec le milieu dans lequel l'espèce biologiquement active recherchée, coexiste avec d'autres espèces. L'affinité sélective apportée par le peptide greffé vis-à-vis de cette espèce recherchée provoquera la complexation de cette dernière sur le peptide greffé sur le polymère de l'électrode ou de la membrane. Cette membrane ou électrode est ensuite retirée du milieu d'analyse et introduite dans un autre milieu, dit de récupération. Dans ce milieu de récupération, contenant un sel support de type NaCl, l'électrode ou membrane est soumise à une oxydation électrochimique, qui provoque l'expulsion de protons, par les groupes acides carboxyliques, jusqu'à un pH suffisant pour la dissociation et le relargage de l'espèce recherchée.

### b) Procédé en continu

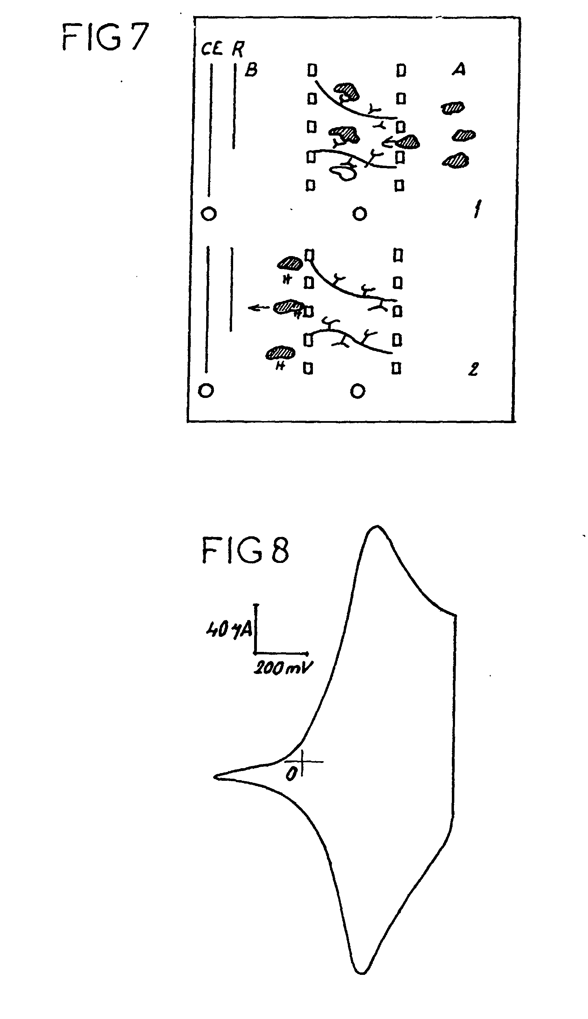

Le copolymère Poly(1, 2), ou le polymère Poly(2) avec sa fonction carboxylique libre, est réalisé sous forme de membrane ou d'électrode, avec éventuellement l'aide d'un polymère support, polycationique ou polyanionique, de type polystyrène sulfonate ou encore membrane perfluorée telle que Nafion. Cette électrode ou membrane, éventuellement composite, constitue la jonction entre deux compartiments A et B, tel que schématisé dans la figure 7. Un exemple d'extraction de carboxypeptidase A par ce dernier procédé en continu est décrit dans ce qui suit.

L'élément biosélectif est constitué, dans le cas exposé de la figure 7, d'un copolymère Poly(1, 2) décrit précédemment, polymérisé dans une membrane de NAFION (Aldrich), obtenue par évaporation sur une grille très fine de platine de 10 µl d'une solution à 5% de NAFION dans un mélange d'alcools linéaires supérieurs (Aldrich). Le film de NAFION qui en résulte, qui présente une épaisseur d'environ 5 µm, sert alors de support pour l'électropolymérisation du copolymère Poly(1, 2), aboutissant à une membrane composite électroactive Poly(1, 2)-NAFION.

Dans une première étape 1, une quantité de 10 grammes de carboxypeptidase A est introduite dans le compartiment A, dans un milieu H₂O-NaCl 0,5 M. Une complexation sur la membrane de composite [Poly(1-2)]-NAFION, s'ensuit immédiatement sur les unités peptidiques de (2), telle que décrite précédemment à l'exemple. 3. Par la suite, une oxydation est réalisée, au moyen d'une contre électrode et électrode de référence introduites dans le compartiment B. Dans une seconde étape 2, l'oxydation électrochimique conduit au relargage de protons dans ce compartiment B, jusqu'à un pH de l'ordre de 4, et au relargage immédiat de la carboxypeptidase A. Une quantité de 1,2 grammes de carboxypeptidase A a été alors récupérée dans le compartiment B lors d'un cycle de ce processus en continu, ainsi que cela a été déterminé par dosage de l'activité enzymatique.
Ces résultats confirment ainsi le phénomène de reconnaissance de ces électrodes vis-à-vis d'enzymes, ainsi que leur capacité d'extraction de ces enzymes. Ce comportement est lié de façon biunivoque à la nature chimique du dipeptide greffé sur la chaîne de polypyrrole.

### Exemple 5: synthèse d'un polypyrrole fonctionnalisé avec un polynucléotide ou oligonucléotide

Cette synthèse est effectuée suivant les 3 étapes suivantes.

### a) Synthèse du monomère [N-hydroxysuccinimide 3-pyrrole]

Dans un tricol sont additionnés 0,4 mole de pyrrole-acide acétique, (I), 30 ml de chloroforme et 0,4 mole de N-hydroxysuccinimide, NHS; (II). Le mélange est soumis à agitation, puis on additionne goutte à goutte une solution de 0,4 mole de dicyclohexylcarbodiimide, DCC, dans 20 ml de chloroforme. Après agitation pendant 2 heures, un solide blanc est formé qui est filtré et lavé au chloroforme. Après évaporation et lavage à l'acétonitrile pour se débarrasser de produits de réaction non nécessaires, on recristallise le produit dans le chloroforme. On obtient alors le composé recherché, [N-hydroxysuccinimide-3-pyrrole], (III), sous forme d'une poudre blanche. 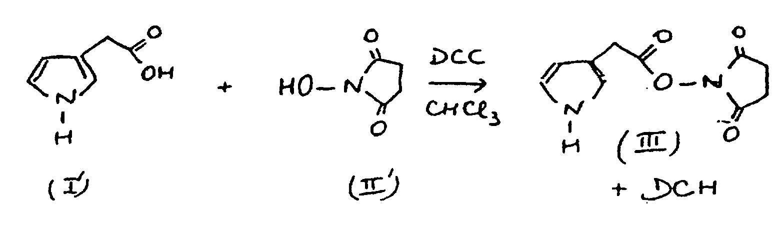 Point de fusion, T_{F} = 135 ° C. ^{1H}RMN (ppm): (NH, 1H, 9, s); pyrrole (2H, 6, 7, m): pyrrole (1H, 6,1, s); CH2 (2H, 3,8, s); Hydroxysuccinimide (4H, 2,8, s).

### b) Synthèse du polymère par électropolymérisation

La solution d'électropolymérisation contient 0,5 M LiClO₄, 0,1 M de monomère (III) dans l'acétonitrile. L'électropolymérisation est réalisée sur électrode de platine de 0,7 cm² de surface, au potentiel de 0,9 V par rapport à une électrode saturée au calomel, ECS, en utilisant une charge d'électropolymérisation de 30 mC. Un film noir apparaît sur l'électrode, correspondant à poly(III), dont l'épaisseur est d'environ 200 nm.

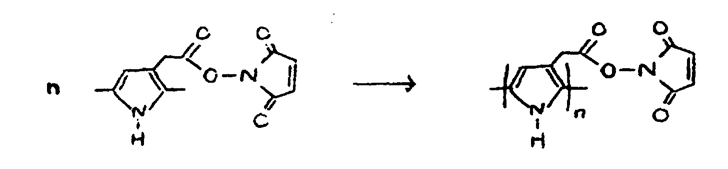

L'électroactivité de ce polymère a été analysée dans un milieu acétonitrile-0,5 M LiClO₄, montrant un pic d'oxydation à 0,28 V/ECS, et un pic de réduction à 0,24 V/ECS, tel que le montre la figure 8. Le faible écart entre ces pics d'oxydation et de réduction, 40 mV, confirme la très grande électroactivité et réversibilité de ce polymère.

### c) Greffage d'un oligonucléotide ODN

L'électrode précédente portant le film de polymère est immergée dans un milieu réactionnel formé d'un mélange de diméthylformamide avec 10 % de tampon borate 0,1 M, à pH de 9,3, et 26,2 nanomoles de l'oligonucléotide, ODN, de 14 bases comportant la séquence CCTAAGAGGGAGTG ainsi qu'une fonction amine en 5' sur laquelle sera opérée la réaction de couplage avec le groupe N-hydroxysuccinimide porté par les unités pyrrole du polymère. Cette réaction de couplage s'effectue en 2 heures. Le greffage s'accompagne également de l'hydrolyse des groupements succinimide, non substitués avec l'oligonucléotide, en acide acétique. Le polymère obtenu sur l'électrode correspond donc à un copolymère Poly([pyrrole-ODN][pyrrole-COOH]).

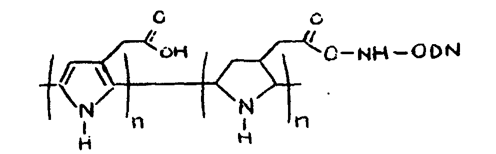

### Exemple 6:. Caractérisation du phénomène de reconnaissance

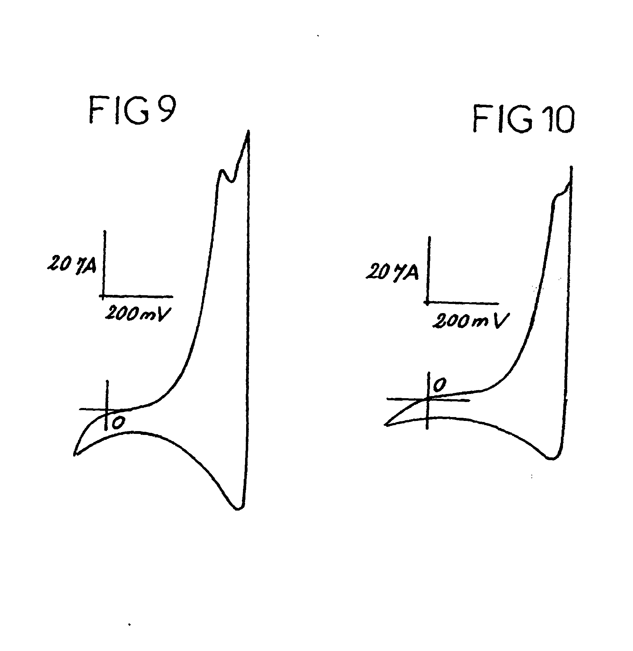

Le phénomène de reconnaissance a été confirmé par la caractérisation électrochimique du film de polymère Poly([Pyrrole-ODN][pyrrole-COOH]), obtenu à l'exemple 5c. L'analyse électrochimique a été menée, d'abord directement sur l'électrode obtenue après synthèse, puis après que cette électrode a été mise en présence d'ODN cible et d'ODN non cible. La réaction d'hybridation de ce polymère a ainsi été réalisée en présence d'ODN complémentaire (cible) et d'ODN non complémentaire (non cible), dans une solution aqueuse tamponnée avec du PEG. L'incubation est effectuée à 37 °C pendant 2 heures, en présence soit d'ODN cible CACTCCCTCTTAGG, en concentration de 335 nanomoles, soit en présence d'ODN non cible, GGTGATAGAAGTATC, en concentration de 272 nanomoles. Après réaction, les films ont été rincés avec le tampon PEG et analysés par voltamètrie cyclique. Les voltamogrammes obtenus sont représentés sur les figures 9 et 10. Les résultats montrent en premier lieu, Figure 9, que le polymère après synthèse, et avant d'être mis en présence de la séquence cible ou non cible, montre un pic d'oxydation réversible qui se situe à 0,34 V/ECS, confirmant l'électroactivité de ce polymère fonctionnalisé. Après réaction d'incubation en présence de la séquence non cible, et après rinçage, le voltamogramme obtenu ne montre aucune modification. Lorsque par contre ce polymère est incubé en présence de la cible, le voltamogramme obtenu, Figure 10, est différent, avec un accroissement du potentiel d'oxydation à 0,40 V/ECS, soit une augmentation de 60 mV. Cet accroissement est tout à fait significatif d'une hybridation qui a eu lieu entre l'ODN et la cible correspondante. Cette augmentation du potentiel d'oxydation est attribuée à un phénomène de complexation du bras pendant le long des chaînes de polypyrrole, s'accompagnant donc d'un accroissement de l'énergie nécessaire à l'oxydation de ce polymère.

Ce résultat confirme que cette nouvelle classe de matériaux électroactifs, de polyhétérocycles conjugués substitués en position 3 par un oligonucléotide, donne effectivement lieu à un phénomène de reconnaissance d'ADN complémentaire, et d'autre part que ces matériaux permettent une lecture électrochimique de cette hybridation sélective. Cette lecture électrochimique ouvre la voie à des génocapteurs de type électrochimique, ampérométrique ou potentiométrique, et d'autre part à des génocapteurs de type transistor microélectrochimique à effet de champ, basés sur une réponse ampérométrique.

## Revendications

1. Polymère conjugué fonctionnalisé, électriquement conducteur et électroactif **caractérisé en ce qu'**il répond à la formule I : 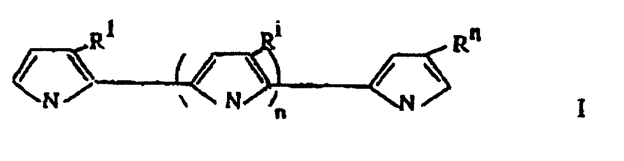 dans laquelle
* n est un nombre entier non nul et i est un nombre entier variant de 2 à n-1, et
* R¹, Rⁱ, Rⁿ, identiques ou différents, représentent H ou un groupe fonctionnel à l'exclusion de CH₂-COOH, susceptible de se lier, par covalence, à une première molécule biologique ou anti-ligand, comprenant au moins un groupement fonctionnel indépendamment choisi dans l'ensemble des groupes fonctionnels suivants :
Yₚ-C-X où X représente OH, un radical O-alkyle inférieur substitué ou non, un halogène, notamment Cl;
Yₚ-NHZ, Z représentant H ou un radical alkyle ;
Yₚ-NH-CO-CF₃ ;
Yₚ-X où X répond à la définition ci-dessus, -Si(alkyle)₃, -Si(alkoxyle)₃, ou un groupement ester activé contenant le N-hydroxy-succinimide, p étant un nombre entier de préférence égal à 1 ou 2,
dans lesquels Y représente un groupement choisi parmi les alkyles ayant de 1 à 5 atome de carbone, les alkoxyles ayant de 1 à 5 atomes de carbone, les polyéthers répondant à la formule générale (CH₂-CH₂-O)ₘ-(CH₂)_{m'}-, m représentant un nombre entier variant de 1 à 3 et m' un nombre entier égal à 1 ou 2.

2. Polymère conjugué, électriquement conducteur, et électroactif comprenant au moins un groupe fonctionnel lié, par covalence, à une première molécule biologique choisie parmi les polynucléotides et les peptides ou anti-ligand, **caractérisé en ce que** ledit polymère répond à la formule II 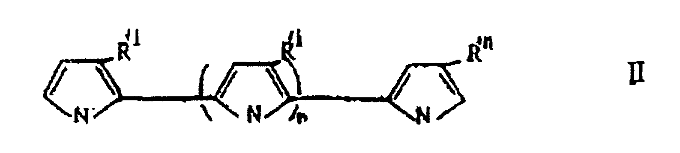 dans laquelle :
* n est un nombre entier non nul et i est un nombre entier variant de 2 à n-1, et
* R'¹, R'¹, R'ⁿ, identiques ou différents, représentent H ou un groupe fonctionnel susceptible de se lier, ou lié, par covalence, à une première molécule biologique ou anti-ligand et dont l'un au moins est un groupe fonctionnel lié, par covalence, à une première molécule biologique ou anti-ligand,
* ledit ou lesdits groupes fonctionnels, liés à une molécule biologique ou ligand étant choisis, avant réaction avec cette dernière, dans l'ensemble des groupes fonctionnels suivant :
Yₚ-C-X où X représente OH, un radical O-alkyle inférieur substitué ou non, un halogène, notamment Cl;
Yₚ-NHZ, Z représentant H ou un radical alkyle ;
Yₚ-NH-CO-CF₃;
Yₚ-X où X répond à la définition ci-dessus, -Si(alkyle)₃, -Si(alkoxyle)₃, ou un groupement ester activé contenant le N-hydroxy-succinimide, p étant un nombre entier de préférence égal à 1 ou 2,
dans lesquels Y représente un groupement choisi parmi les alkyles ayant de 1 à 5 atome de carbone, les alkoxyles ayant de 1 à 5 atomes de carbone, les polyéthers répondant à la formule générale (CH₂-CH₂-O)ₘ-(CH₂)_{m'}-, m représentant un nombre entier variant de 1 à 3 et m' un nombre entier égal à 1 ou 2, ou
-CH2-COOH ; à la condition que les groupes fonctionnels liés à une molécule biologique choisie parmi les polynucléotides et les peptides sont identiques s'ils consistent, avant réaction avec la première molécule biologique, en -(CH₂)-COOH-.

3. Polymère conjugué selon la revendication 2, **caractérisé en ce que** la ou les premières molécules biologiques ou anti-ligands sont choisis parmi les peptides ou dérivés de peptides notamment Gly-Phe, Phe-Pro, Phe-Hea-Pro, et parmi les polynucléotides tels que l'oligonucléotide de séquence: CCTAAGAGGGAGTG.

4. Utilisation d'un polymère conjugué tel que défini dans l'une des revendications 2 à 3, pour détecter ou doser, in vitro ou in vivo, une seconde molécule biologique ou ligand, différente de l'anti-ligand et interagissant spécifiquement avec ce dernier, la détection et/ou le dosage dudit ligand étant effectué par observation et/ou mesure d'une différence de potentiel ou d'une variation de courant entre le polymère conjugué non lié au ligand et le polymère conjugué lié au ligand.

5. Utilisation, selon la revendication 4, d'un polymère défini à la revendication 3, pour détecter et/ou doser une enzyme, telle qu'une enzyme protéolytique notamment la carboxypeptidase A.

6. Utilisation selon la revendication 4, d'un polymère défini à l'une des revendications 2 à 5, pour détecter et/ou doser un polynucléotide tel que l'oligonucléotide de séquence CACTCCCTCTTAGG.

7. Utilisation d'un polymère conjugué selon l'une quelconque des revendications 2 à 3, pour extraire, in vitro ou in vivo, une seconde molécule biologique ou ligand, différente de l'anti-ligand et interagissant spécifiquement avec ce dernier.

8. Utilisation d'un polymère conjugué selon l'une quelconque des revendications 2 à 3, **caractérisé en ce que** le polymère conjugué est déposé sur un substrat conducteur, tel que le métal ou un dérivé du carbone, ou sous la forme d'un film auto-supporté.

9. Electrode **caractérisée en ce qu'**elle est constituée d'un substrat conducteur tel que un métal ou un dérivé de carbone et d'un polymère tel que défini dans l'une quelconque des revendications 2 à 3.

10. Film auto supporté d'un polymère **caractérisé en ce qu'**il est constitué d'un polymère tel que défini dans l'une quelconque des revendications 2 à 3.

## Patentansprüche

1. Funktionalisiertes elektrisch leitfähiges und elektroaktives konjugiertes Polymer, **dadurch gekennzeichnet, dass** es Formel I entspricht: 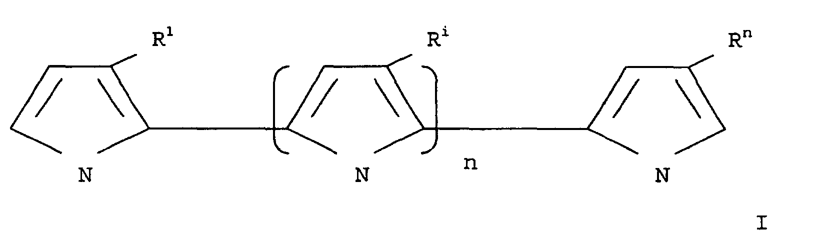 in der
* n eine ganze Zahl ungleich Null, und i eine ganze Zahl, die von 2 bis n-1 variiert, ist, und
* R¹, Rⁱ, Rⁿ, die identisch oder unterschiedlich sind, ein H oder eine funktionelle Gruppe, mit Ausnahme von CH₂-COOH, repräsentieren, die über Kovalenz an ein erstes biologisches Molekül oder einen Anti-Liganden binden kann, und die zumindest eine funktionelle Gruppe aufweisen, die unabhängig ausgewählt ist aus der Gesamtheit der nachfolgenden funktionellen Gruppen:
Yₚ-C-X, wobei X ein OH, ein substituiertes oder nichtsubstituiertes O-Niederalkyl-Radikal, ein Halogen, insbesondere Cl, repräsentiert;
Yₚ-NHZ, wobei Z ein H oder ein Alkyl-Radikal repräsentiert;
Yₚ-NH-CO-CF₃;
Yₚ-X, wobei X der obigen Definition entspricht, -Si(alkyl)₃, -Si(alkoxyl)₃, oder eine aktivierte Estergruppe, die N-Hydroxysuccinimid enthält, wobei p eine ganze Zahl von vorzugsweise 1 oder 2 ist,
in denen Y eine Gruppe repräsentiert, die ausgewählt ist aus den Alkylen mit 1 bis 5 Kohlenstoffatomen, den Alkoxylen mit 1 bis 5 Kohlenstoffatomen, den Polyethern, die der allgemeinen Formel (CH₂-CH₂-O)ₘ-(CH₂)_{m'} entsprechen, wobei m eine ganze Zahl, die von 1 bis 3 variiert, und m' eine ganze Zahl von 1 oder 2 repräsentieren.

2. Elektrisch leitfähiges und elektroaktives konjugiertes Polymer, das zumindest eine funktionelle Gruppe aufweist, die über Kovalenz an ein erstes biologisches Molekül gebunden ist, das ausgewählt ist aus den Polynukleotiden und den Peptiden oder dem Anti-Liganden, **dadurch gekennzeichnet, dass** das Polymer Formel II entspricht: 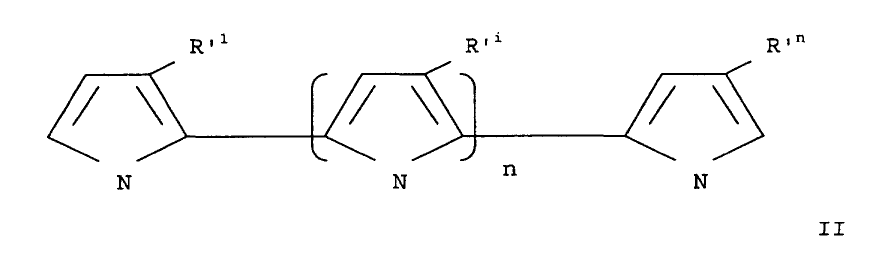 in der:
* n eine ganze Zahl ungleich Null, und i eine ganze Zahl, die von 2 bis n-1 variiert, ist, und
* R'¹, R'ⁱ, R'ⁿ, die identisch oder unterschiedlich sind, ein H oder eine funktionelle Gruppe repräsentieren, die über Kovelenz an ein erstes biologisches Molekül oder einen Anti-Liganden binden kann oder daran gebunden ist, und von denen zumindest eine Gruppe eine funktionelle ist, die über Kovalenz an ein erstes biologisches Molekül oder einen Anti-Liganden gebunden ist,
* die eine oder mehreren funktionellen Gruppen, die an ein biologisches Molekül oder einen Liganden gebunden sind, vor der Reaktion mit letzteren aus der Gesamtheit der folgenden funktionellen Gruppen ausgewählt werden:
Yₚ-C-X, wobei X ein OH, ein substituiertes oder nichtsubstituiertes O-Niederalkyl-Radikal, ein Halogen, insbesondere Cl, repräsentiert;
Yₚ-NHZ, wobei Z ein H oder ein Alkyl-Radikal repräsentiert;
Yₚ-NH-CO-CF₃;
Yₚ-X, wobei X der obigen Definition entspricht, -Si(alkyl)₃, -Si(alkoxyl)₃, oder eine aktivierte Estergruppe, die N-Hydroxysuccinimid enthält, wobei p eine ganze Zahl von vorzugsweise 1 oder 2 ist,
in denen Y eine Gruppe repräsentiert, die ausgewählt ist aus den Alkylen mit 1 bis 5 Kohlenstoffatomen, den Alkoxylen mit 1 bis 5 Kohlenstoffatomen, den Polyethern, die der allgemeinen Formel (CH₂-CH₂-O)ₘ-(CH₂)ₘ, entsprechen, wobei m eine ganze Zahl, die von 1 bis 3 variiert, und m' eine ganze Zahl von 1 oder 2, oder -CH₂-COOH repräsentieren; unter der Bedingung, dass die funktionellen Gruppen, die an ein biologisches Molekül gebunden sind, das ausgewählt ist aus den Polynukleotiden und den Peptiden, identisch sind, wenn sie vor der Reaktion mit dem ersten biologischen Molekül aus -(CH₂)-COOH bestehen.

3. Konjugiertes Polymer nach Anspruch 2, **dadurch gekennzeichnet, dass** das oder die ersten biologischen Moleküle oder Anti-Liganden ausgewählt sind aus den Peptiden oder Peptidderivaten, insbesondere aus Gly-Phe, Phe-Pro, Phe-Hea-Pro, und aus den Polynukleotiden, wie dem Oligonukleotid mit der Sequenz: CCTAAGAGGGAGTG.

4. Verwendung eines wie in einem der Ansprüche 2 bis 3 definierten konjugierten Polymers, um ein zweites biologisches Molekül oder einen Liganden in vitro oder in vivo zu detektieren oder zu bestimmen, der sich von dem Anti-Liganden unterscheidet und spezifisch mit diesem interagiert, wobei die Detektion und/oder die Bestimmung dieses Liganden durch Erfassung und/oder Messung einer Potenzialdifferenz oder einer Stromveränderung zwischen dem nicht an den Liganden gebundenen konjugierten Polymer und dem an den Liganden gebundenen Polymer erfolgt.

5. Verwendung eines in Anspruch 4 definierten Polymers gemäß Anspruch 3, um ein Enzym, wie bspw. ein proteolytisches Enzym, insbesondere Carboxypeptidase A, zu detektieren und/oder zu bestimmen.

6. Verwendung eines in einem der Ansprüche 2 bis 5 definierten Polymers gemäß Anspruch 4, um ein Polynukleotid, wie bspw. das Oligonukleotid mit der Sequenz CACTCCCTCTTAGG, zu detektieren und/oder zu bestimmen.

7. Verwendung eines konjugierten Polymers nach Anspruch 2 oder 3, um ein zweites biologisches Molekül oder einen Liganden in vitro oder in vivo zu extrahieren, der sich von dem Anti-Liganden unterscheidet und mit letzterem spezifisch interagiert.

8. Verwendung eines konjugierten Polymers nach Anspruch 2 oder 3, **dadurch gekennzeichnet, dass** das konjugierte Polymer auf einem leitfähigen Substrat, wie bspw. Metall oder ein Kohlenstoffderivat oder in Form eines selbsttragenden Films, aufgebracht wird.

9. Elektrode, **dadurch gekennzeichnet, dass** sie aus einem leitfähigen Substrat, wie bspw. Metall oder ein Kohlenstoffderivat, und einem wie in Anspruch 2 oder 3 definierten Polymer gebildet ist.

10. Selbsttragender Film aus einem Polymer, **dadurch gekennzeichnet, dass** er aus einem wie in Anspruch 2 oder 3 definierten Polymer gebildet ist.

## Claims

1. Electrically conductive, electroactive functionalized conjugated polymer, **characterized in that** it corresponds to the formula I: 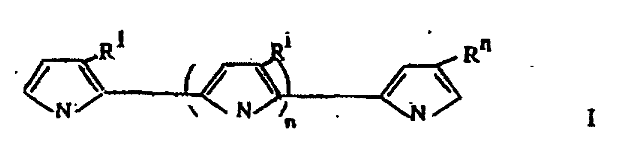 in which:
* n is a non-zero integer and i is an integer ranging from 2 to n-1, and
* R¹, Rⁱ and Rⁿ, which may be identical or different, represent H or a functional group with the exception of CH₂-COOH, capable of covalently bonding with a first biological molecule or antiligand, comprising at least one functional group independently chosen from the following set of functional groups:
Yₚ-C-X where X represents OH, a substituted or unsubstituted lower O-alkyl radical, or a halogen, in particular Cl;
Yₚ-NHZ, Z representing H or an alkyl radical;
Yₚ-NH-CO-CF₃;
Y_{P}-X where X corresponds to the above definition, -Si(alkyl)₃, -Si(alkoxyl)₃ or an activated ester group containing N-hydroxysuccinimide, p being an integer preferably equal to 1 or 2,
in which Y represents a group chosen from alkyls having from 1 to 5 carbon atoms, alkoxyls having from 1 to 5 carbon atoms, and polyethers corresponding to the general formula (CH₂-CH₂-O)ₘ-(CH₂)_{m'}-, m representing an integer ranging from 1 to 3 and m' an integer equal to 1 or 2.

2. Electrically conductive, electroactive conjugated polymer comprising at least one functional group which is covalently bonded to a first biological molecule chosen from among polynucleotides and peptides or antiligand, **characterized in that** said polymer corresponds to the formula II 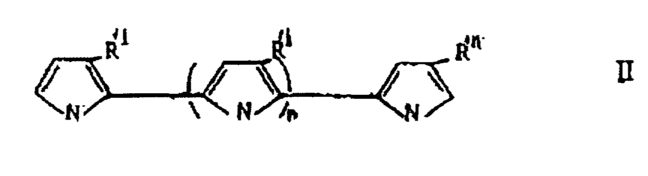 in which:
* n is a non-zero integer and i is an integer ranging from 2 to n-1, and
* R^{'1}, R^{'i} and R^{'n}, which may be identical or different, represent H or a functional group capable of covalently bonding with, or covalently bonded to, a first biological molecule or antiligand and at least one of which is a functional group which is covalently bonded to a first biological molecule or antiligand,
* said functional group or groups bonded to a biological molecule or ligand being chosen, before reaction with the latter, from the following set of functional groups:
Yₚ-C-X where X represents OH, a substituted or unsubstituted lower O-alkyl radical, or a halogen, in particular Cl;
Yₚ-NHZ, Z representing H or an alkyl radical;
Yₚ-NH-CO-CF₃;
Yₚ-X where X corresponds to the above definition, -Si(alkyl)₃, -Si(alkoxyl)₃ or an activated ester group containing N-hydroxysuccinimide, p being an integer preferably equal to 1 or 2,.
in which Y represents a group chosen from alkyls having from 1 to 5 carbon atoms, alkoxyls having from 1 to 5 carbon atoms, and polyethers corresponding to the general formula (CH₂-CH₂-O)ₘ-(CH)_{m'}-, m representing an integer ranging from 1 to 3 and m' an integer equal to 1 or 2, or
-CH₂-COOH; provided that the functional groups bonded to a biological molecule chosen from polynucleotides and peptides are identical if they consist, before reaction with the first biological molecule, of -(CH₂)-COOH.

3. Conjugated polymer according to Claim 2, **characterized in that** the first biological molecule or molecules or antiligands are chosen from peptides or peptide derivatives and in particular Gly-Phe, Phe-Pro and Phe-Hea-Pro, and from polynucleotides such as the oligonucleotide of sequence: CCTAAGAGGGAGTG.

4. Use of a conjugated polymer as defined in one of Claims 2 to 3, for detecting or assaying, in vitro or in vivo, a second biological molecule or ligand, which is different from the antiligand and which interacts specifically with the latter, said ligand being detected and/or assayed by observation and/or measurement of a potential difference or of a variation in current between the conjugated polymer not bonded to the ligand and the conjugated polymer bonded to the ligand.

5. Use according to Claim 4, of a polymer defined in Claim 3, for detecting and/or assaying an enzyme, such as a proteolytic enzyme, in particular carboxypeptidase A.

6. Use according to Claim 4, of a polymer defined in one of Claims 2 to 5, for detecting and/or assaying a polynucleotide such as the oligonucleotide of sequence: CACTCCCTCTTAGG.

7. Use of a conjugated polymer according to either of Claims 2 and 3, in order to extract, in vitro or in vivo, a second biological molecule or ligand, which is different from the antiligand and which interacts specifically with the latter.

8. Use of a conjugated polymer according to either of Claims 2 and 3, **characterized in that** the conjugated polymer is deposited on a conductive substrate, such as metal or a carbon derivative, or is in the form of a self-supporting film.

9. Electrode, **characterized in that** it consists of a conductive substrate such as a metal or a carbon derivative and of a polymer as defined in either of Claims 2 and 3.

10. Self-supporting film of a polymer, **characterized in that** it consists of a polymer as defined in either of Claims 2 and 3.
